# EUROPEAN PATENT SPECIFICATION

(11) **EP 2 454 373 B1**
(45) Date of publication and mention of the grant of the patent: **19.11.2014**
(21) Application number: 10740787.6
(22) Date of filing: 15.07.2010
(51) Int. Cl.: C12N 15/67

(54) **ENHANCEMENT OF CELLULAR PRODUCTION THROUGH MECHANOTRANSDUCTION**
ERHÖHTE ZELLPRODUKTION DURCH MECHANOTRANSDUKTION
AUGMENTATION DE LA PRODUCTION CELLULAIRE PAR TRANSDUCTION MECANIQUE

(30) Priority: 15.07.2009 US 225694 P
(43) Date of publication of application: 23.05.2012
(73) Proprietor: AbbVie Inc., North Chicago, IL 60064 (US)
(72) Inventor: PIPARIA, Reema, Worcester MA 01605 (US); CORREIA, Ivan, Winchester MA 01890 (US)
(74) Representative: Modiano, Micaela Nadia
(86) International application number: PCT/US2010/042143
(87) International publication number: WO 2011/008959

(56) References cited:
- WO-A1-98/45468
- SENGER RYAN S ET AL: "Effect of shear stress on intrinsic CHO culture state and glycosylation of recombinant tissue-type plasminogen activator protein." BIOTECHNOLOGY PROGRESS, vol. 19, no. 4, July 2003 (2003-07), pages 1199-1209, XP002600489 ISSN: 8756-7938
- GUO ET AL: "Ultrasound-targeted microbubble destruction improves the low density lipoprotein receptor gene expression in HepG2 cells" BIOCHEMICAL AND BIOPHYSICAL RESEARCH COMMUNICATIONS, ACADEMIC PRESS INC. ORLANDO, FL, US LNKD- DOI:10.1016/J.BBRC.2006.02.179, vol. 343, no. 2, 5 May 2006 (2006-05-05), pages 470-474, XP005345814 ISSN: 0006-291X
- SMITH R C ET AL: "Spatial and temporal control of transgene expression through ultrasound-mediated induction of the heat shock protein 70B promoter in vivo" HUMAN GENE THERAPY, MARY ANN LIEBERT, NEW YORK ,NY, US LNKD- DOI:10.1089/104303402317322267, vol. 13, no. 6, 10 April 2002 (2002-04-10) , pages 697-706, XP002344533 ISSN: 1043-0342
- LUDWIG A ET AL: "Shear stress induced variation of cell condition and productivity" JOURNAL OF BIOTECHNOLOGY, ELSEVIER SCIENCE PUBLISHERS, AMSTERDAM, NL LNKD- DOI:10.1016/0168-1656(93)90110-9, vol. 27, no. 2, 1 January 1993 (1993-01-01), pages 217-223, XP023788218 ISSN: 0168-1656 [retrieved on 1993-01-01]
- ZIROS PANOS G ET AL: "The bone-specific transcriptional regulator Cbfa1 is a target of mechanical signals in osteoblastic cells" JOURNAL OF BIOLOGICAL CHEMISTRY, vol. 277, no. 26, 28 June 2002 (2002-06-28), pages 23934-23941, XP002600490 ISSN: 0021-9258
- NAKAMURA T ET AL: "Mechanical strain and dexamethasone selectively increase surfactant protein C and tropoelastin gene expression." AMERICAN JOURNAL OF PHYSIOLOGY. LUNG CELLULAR AND MOLECULAR PHYSIOLOGY MAY 2000 LNKD- PUBMED:10781428, vol. 278, no. 5, May 2000 (2000-05), pages L974-L980, XP002600491 ISSN: 1040-0605
- INGBER DONALD E: "Tensegrity I. Cell structure and hierarchical systems biology." JOURNAL OF CELL SCIENCE, vol. 116, no. 7, 1 April 2003 (2003-04-01) , pages 1157-1173, XP002600492 ISSN: 0021-9533
- INGBER D E: "Tensegrity: the architectural basis of cellular mechanotransduction." ANNUAL REVIEW OF PHYSIOLOGY 1997 LNKD- PUBMED:9074778, vol. 59, 1997, pages 575-599, XP002600493 ISSN: 0066-4278

## Description

### BACKGROUND OF THE INVENTION

Chinese Hamster Ovary (CHO) cells are the most commonly used mammalian host cell line for producing recombinant proteins in the biopharmaceutical industry. Cell culture processes using mammalian cells, including CHO cells, for producing bio-therapeutics are faced with a number of challenges including compressed product development time lines, capacity shortages and limitations on the proliferation and productivity of cell lines.

The present invention addresses these issues by applying a tensegrity model to mammalian cells, such as CHO cells. By applying tensegrity forces to such cells, the level of recombinant antibody produced by the cells can be increased.

### SUMMARY OF THE INVENTION

The present invention is directed to methods for increasing production of an antibody of interest from a host cell comprising subjecting said cell to a tensegrity force, wherein the tensegrity force is applied to the cell in an amount effective to increase said antibody production from the cell. The methods of the invention are directed to increasing production of a target antibody from cells and/or cell culture. The tensegrity forces can be stress that is applied to the cells, and can include one or more of the following: mechanical stress, shear stress, stretch effects and pressure induced stress, for example, pressure induced by sound waves. In certain embodiments, the antibody is an IgG. In certain embodiments, the cells are Chinese hamster ovary (CHO) cells.

In certain embodiments, the cells are free-floating in a culture. In alternative embodiments, the cells are adherent cells capable of adhering to a substrate.

In certain embodiments, the present invention is directed to methods of increasing production of a recombinant antibody from cells of a cell culture by applying a stress to the cells. In certain embodiments, the antibody is an IgG. In certain embodiments, the cells are Chinese hamster ovary (CHO) cells.

In certain embodiments of the present invention, the cells undergoing stress are cultured in a commercial bioreactor.

One method of the present invention involves subjecting cells expressing an antibody of interest to a controlled mechanical shear stress. In certain embodiments, shear stress (torque) can be applied to the surface of a cell using membrane-bound ferromagnetic beads coated with antibodies that can adhere to the cytoskeleton of the cells. The beads can be magnetized in one direction by applying a weaker twisting magnetic field.

Alternative methods of the present invention involve subjecting cells that express an antibody of interest to a biomechanical culture system capable of subjecting adherent cells to diverse biaxial stress/strain culture conditions. In certain embodiments, the biomechanical culture system is traction microscopy. In certain embodiments of the invention, the biomechanical culture system permits live microscopic imaging of the cells.

In certain embodiments, the viability of cells undergoing stress can be measured. Non-limiting examples of such cell viability assays include, but are not limited to, dye uptake assays (*e.g.,* calcein AM assays), XTT cell viability assays, and dye exclusion assays (*e.g.,* trypan blue, Eosin, or propidium dye exclusion assays).

In certain embodiments, the amount of antibody secreted by cells undergoing stress can be assayed. In certain embodiments, the antibody is an IgG, which is released in the media that forms the cells' microenvironment.

In certain embodiments, the behavior of cells undergoing stress can be assayed. Such cell behavior includes, but is not limited to, cell migration and morphology. In certain embodiments, microscopic techniques, such as, but not limited to, scanning electron microscopy, phase contrast microscopy, and/or transmission electron microscopy can be used to evaluate the changes in cell behavior over time.

### BRIEF DESCRIPTIONS OF THE DRAWING

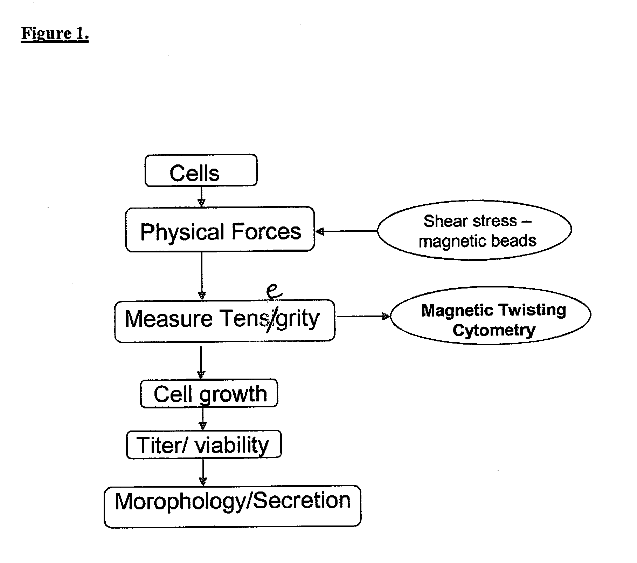

**Figure 1** Flow chart describing the sequence of experimental procedures to study the tensegrity model in CHO cells.

### DETAILED DESCRIPTION OF THE INVENTION

The present invention is directed to methods of increasing antibody production via the application of tensegrity forces on cells and cell cultures. The tensegrity forces can be stress that is applied to the cells, and can include one or more of the following: mechanical stress, shear stress, stretch effects, and pressure induced stress, for example, ultrasound induced stress.

For clarity, and not by way of limitation, this detailed description is divided into the following sub-portions:
1. Methods of Applying Tensegrity Forces;
2. Modulation of Target Protein Production via Application of Tensegrity Forces; and
3. Large Scale Production Incorporating Tensegrity Force Optimization.

### 1. Methods of Applying Tensegrity Forces

The present invention is directed to methods of increasing antibody production by applying tensegrity forces to cells or cell cultures. In the cellular tensegrity model, tensional forces are borne by cytoskeletal microfilaments and intermediate filaments, and these forces are balanced by interconnected structural elements that resist compression, most notably, internal microtubule struts and extracellular matrix (ECM) adhesions. (See, e.g., Ingber, D.E., Tensegrity: the architectural basis of cellular mechanotransduction. Annu Rev Physiol, 1997. 59: p. 575-99; Ingber, D.E., Tensegrity II. How structural networks influence cellular information processing networks. J Cell Sci, 2003. 116(Pt 8): p. 1397-408; Ingber, D.E., Tensegrity I. Cell structure and hierarchical systems biology. J Cell Sci. 2003 Apr 1;116(Pt 7):1157-73; and Wang, N., J.D. Tytell, and D. Ingber, Mechanotransduction at a distance: mechanically coupling the extracellular matrix with nucleus. Molecular cell biology, January 2009. 10: p. 75-82).. Such tensegrity forces can include force that stress, for example, mechanically stress the target cells or cell cultures.

In certain embodiments, tensegrity forces are received and the signal is transduced into a cell via a complementary force balance between tensed microfilaments, compressed microtubules and transmembrane integrin receptors in living cells. When force is applied to integrins, thermodynamic and kinetic parameters can change locally for cytoskeleton-associated molecules that physically experience the mechanical load. When force is applied to non-adhesion receptors that do not link to the cytoskeleton, stress dissipates locally at the cell surface, and the biochemical response is muted. In one non-limiting example, when tension is applied to integrins, new tubulin monomers add onto the end of a microtubule, and the microtubule is decompressed as a result of a change in the critical concentration of tubulin. In certain embodiments, molecules that are physically distorted by stress transferred from integrins to the cytoskeleton can change their kinetics, for example, increasing their rate constant for chemical conversion of a substrate into a product. In this manner, both cytoskeletal structure (architecture) and prestress (tension) in the cytoskeleton can modulate the cellular response to mechanical stress.

In certain embodiments, force application through the cytoskeleton can be transduced to nuclear pores of a cell and stretch the pore, opening the baskets and other components of the inner nuclear pore complex, alter the pore's opening kinetics or modulate its molecular composition thereby increasing nuclear transport. Such an effect can influence post-transcriptional control of gene expression.

In certain embodiments, the tensegrity force can be a controlled mechanical shear stress. In certain embodiments, the shear stress can be applied directly to the cell surface. Shear stress (torque) can be applied to the surface using membrane-bound magnetic beads, for example, ferromagnetic beads, coated with antibodies that adhere to the cytoskeleton of the cell. The beads can be magnetized in one direction by applying a twisting magnetic field. Electron microscopy images can be taken to show how ferromagnetic beads attach to the surface of the cells.

The shear stress is applied to the cells in an amount effective to increase the level of antibody production from the cells.

In certain embodiments, the ferromagnetic beads are between about 0.25 and 0.5 µm in diameter, between about 0.5 and 0.75 µm in diameter, between about 0.75 and 1 µm in diameter, between about 1 and 2 µm in diameter, between about 2 and 3 µm in diameter, between about 3 and 4 µm in diameter, between about 4 and 5 µm in diameter, between about 5 and 6 µm in diameter, between about 6 and 7 µm in diameter, between about 7 and 8 µm in diameter, between about 8 and 9 µm in diameter, or between about 9 and 10 µm in diameter. In certain embodiments, the ferromagnetic beads are between about 1-6 µm in diameter.

In certain embodiments, the cells are CHO cells, and the ferromagnetic beads are coated with anti-CHO antibodies.

Cellular deformation that results in response to stress application can be determined using magnetic twisting cytometry (MTC), which is a technique used to exert mechanical stresses on living cells by first magnetizing and then rotating the ferromagnetic beads that are bound to the surface of cells. In certain embodiments, the MTC device can consist of seven major components:(i) a high-voltage generator to provide the current to magnetize the beads; (ii) one [for one-dimensional (1D) MTC] bipolar current sources for twisting the beads; (iii) a computer for controlling the twisting apparatus; (iv) an inverted microscope for observing the sample; (v) a charge-coupled device (CCD) camera that uses software capable of synchronizing image capture with step function or oscillatory wave magnetic fields; (vi) a device to maintain the correct temperature of the cultured cells; and (vii) a microscope insert that holds the sample and contains two pairs of coils for 1D MTC that generate the alternating electric fields used to magnetize and twist the beads.

In certain embodiments, the tensegrity force can be generated by a biomechanical culture system capable of subjecting cells to diverse biaxial stress/strain. In one non-limiting example, the biomechanical culture system can be traction microscopy. For example, cells can be attached to a substrate, for example, but not limited to, silicone, hydrogel, polyacrylamide, laminin, or elastin. The substrate can be further coated with collagen. Microbeads, for example, fluorescent microbeads, can be embedded near the apical surface of the substrate to trace the dynamics of the cytoskeleton network under the microscope. In certain embodiments, the microbeads are at least 0.001 µm diameter, at least 0.005 µm diameter, at least 0.01 µm diameter, at least 0.05 µm diameter. at least 0.1 µm diameter, at least 0.15 µm diameter, at least 0.2 µm diameter, at least 0.25 µm diameter, at least 0.3 µm diameter, at least 0.35 µm diameter, at least 0.4 µm diameter, at least 0.45 µm diameter or at least 0.5 µm diameter. In certain embodiments, the microbeads are 0.2 µm diameter.

Stiffness and the stretch are two variables that can be controlled in the system. For example, stiffness can be controlled by adjusting the concentration of the substrate, for example, a hydrogel substrate, and the stretch forces can be controlled by the magnitude of the forces applied to the substrate. The substrate can be stretched along an axis. For example, the substrate can be stretched symmetrically along two orthogonal axes using computer controlled stepper motors. The stretch and stiffness are applied to the cells in an amount effective to increase the level of antibody production from the cells.

In certain embodiments, the cells are cultured on substrate in a cell culture plate, for example, a multi-well cell culture plate.

In certain embodiments, tensegrity forces can be generated by applying sound pressure to cells or cell culture, for example, ultrasound waves, or any other high or low frequency sound waves, which may optionally be provided in a cyclic manner. Such sound pressure can change properties of the cells. For example, at low sound frequencies CHO cells subjected to cyclic sound stress can alter their expression and/or secretory pathway and increase antibody production levels. In certain embodiments, the cyclic sound pressure is applied to the cells in an amount effective to increase the level of antibody production from the cells.

In certain embodiments, the viability and morphology of cells subjected to tensegrity forces can be monitored. Cell viability can be determined using dyes that can permeate the membranes of cells. For example, membrane permeable fluorescent cell markers, such as calcein AM, a green fluorescent cell marker, can be used to monitor cell viability. Calcein AM is membrane-permeable and can be introduced into cells via incubation. Once inside the cells, Calcein AM is hydrolyzed by endogenous esterase into the highly negatively charged green fluorescent calcein. The fluorescent calcein is retained in the cytoplasm in live cells.

Dye exclusion tests can also be used to determine the number of viable cells present in a cell suspension or cell culture. Live cells possess intact cell membranes that exclude certain dyes, for example, but not limited to, trypan blue, Eosin, and propidium, whereas dead cells do not. In a dye exclusion test, a cell suspension or culture can be mixed with dye and visually examined to determine whether cells take up or exclude dye. The cells can be counted using, for example, a hemacytometer mounted on an inverted light microscope. Viable cells have a clear cytoplasm whereas a nonviable cell have cytoplasm marked by the dye.

In certain embodiments, cell viability can be determined by measuring the activity of enzymes in living cells. Methods of determining cell viability can also be used to determine the rate of cell proliferation. For example, the activity of mitochondrial enzymes, which are inactivated shortly after cell death, can be measured to determine cell viability. In certain embodiments, mitochondrial enzyme activity can be measured with an XTT Cell Viability Assay Kit. XTT is a tetrazolium derivative. Mitochondria enzymes in live cells reduce XTT to a highly water-soluble orange colored product. The amount of water-soluble product generated from XTT is proportional to the number of living cells in a sample and can be quantified by measuring absorbance at wavelength of 475 nm.

Cell migration and morphology are consequences of changes in underlying cytoskeletal organization and dynamics. The migration and morphology of cells subjected to tensegrity forces can be monitored using microscopic techniques to evaluate changes in cell migration and morphology over time. Such microscopic techniques include, but are not limited to, scanning electron microscopy, phase contrast microscopy, and transmission electron microscopy.

In certain embodiments, the level of antibody secreted by cells or cell culture subjected to tensegrity forces can be measured to determine increases in antibody production. In particular, the level of antibody secreted by cells into their microenvironment can be measured. The amount of secreted antibody can be determined, for example, by contacting a solution containing the antibodies to an antibody binding substance bound to the surface of a substrate. The amount of bound antibody can the be quantified. For example, secreted IgG antibodies can be measured by contacting the antibody to a Protein A ligand covalently bound to the surface of a macroporous polymer resin. Cells can be centrifuged and the supernatant collected can be injected onto a Protein A column, whereby IgG in the sample is captured. Bound IgG can then be eluted by lowering the pH and detecting the eluted material directly at 280nm.

In certain embodiments, once a solution or mixture comprising an antibody has been obtained, separation of the antibody from the other proteins produced by the cell, can be performed using a combination of different purification techniques, including ion exchange separation step(s) and hydrophobic interaction separation step(s). The separation steps separate mixtures of proteins on the basis of their charge, degree of hydrophobicity, or size. In certain embodiments, separation is performed using chromatography, including cationic, anionic, and hydrophobic interaction. Several different chromatography resins are available for each of these techniques, allowing accurate tailoring of the purification scheme to the particular protein involved. The essence of each of the separation methods is that proteins can be caused either to traverse at different rates down a column, achieving a physical separation that increases as they pass further down the column, or to adhere selectively to the separation medium, being then differentially eluted by different solvents. In some cases, the antibody is separated from impurities when the impurities specifically adhere to the column and the antibody does not, *i.e*., the antibody is present in the flow through.

In certain embodiments, separation steps are employed to separate an antibody from one or more host cell proteins. In certain embodiments, the purification strategies exclude the use of Protein A affinity chromatography, for example purification of IgG₃ antibodies, as IgG₃ antibodies bind to Protein A inefficiently. Other factors that allow for specific tailoring of a purification scheme include, but are not limited to: the presence or absence of an Fc region (*e.g.*, in the context of full length antibody as compared to an Fab fragment thereof) because Protein A binds to the Fc region; the particular germline sequences employed in generating the antibody of interest; and the amino acid composition of the antibody (*e.g.,* the primary sequence of the antibody as well as the overall charge/hydrophobicity of the molecule). Antibodies sharing one or more characteristic can be purified using purification strategies tailored to take advantage of that characteristic.

### 2. Modulation of Target Antibody Production via Application of Tensegrity Forces

In the methods of the invention, the target protein is an antibody. Applying tensegrity forces to the cells or cell culture increases production of the target antibody, e.g., a recombinantly expressed antibody, by the cells or cell culture.

The term "antibody" as used in this section refers to an intact antibody or an antigen binding fragment thereof.

In certain embodiments, the cells and cell culture express and secrete the antibody ABT-874.

The antibodies of the present disclosure can be generated by a variety of techniques, including immunization of an animal with the antigen of interest followed by conventional monoclonal antibody methodologies *e.g.*, the standard somatic cell hybridization technique of Kohler and Milstein (1975) Nature 256: 495. Although somatic cell hybridization procedures are preferred, in principle, other techniques for producing monoclonal antibody can be employed *e.g.*, viral or oncogenic transformation of B lymphocytes.

One preferred animal system for preparing hybridomas is the murine system. Hybridoma production is a very well-established procedure. Immunization protocols and techniques for isolation of immunized splenocytes for fusion are known in the art. Fusion partners (*e.g.*, murine myeloma cells) and fusion procedures are also known.

An antibody preferably can be a human, a chimeric, or a humanized antibody. Chimeric or humanized antibodies of the present disclosure can be prepared based on the sequence of a non-human monoclonal antibody prepared as described above. DNA encoding the heavy and light chain immunoglobulins can be obtained from the non-human hybridoma of interest and engineered to contain non-murine (*e.g.*, human) immunoglobulin sequences using standard molecular biology techniques. For example, to create a chimeric antibody, murine variable regions can be linked to human constant regions using methods known in the art (see *e.g.,* U.S. Patent No. 4,816,567 to Cabilly et al.). To create a humanized antibody, murine CDR regions can be inserted into a human framework using methods known in the art (see *e.g.,* U.S. Patent No. 5,225,539 to Winter, and U.S. Patent Nos. 5,530,101; 5,585,089; 5,693,762 and 6,180,370 to Queen et al.).

To express an antibody of the present disclosure, DNAs encoding partial or full-length light and heavy chains are inserted into one or more expression vector such that the genes are operatively linked to transcriptional and translational control sequences. (See, *e.g.,* U.S. Pat. No. 6,914,128).

In this context, the term "operatively linked" is intended to mean that an antibody gene is ligated into a vector such that transcriptional and translational control sequences within the vector serve their intended function of regulating the transcription and translation of the antibody gene. The expression vector and expression control sequences are chosen to be compatible with the expression host cell used, for example CHO cells. The antibody light chain gene and the antibody heavy chain gene can be inserted into a separate vector or, more typically, both genes are inserted into the same expression vector. The antibody genes are inserted into an expression vector by standard methods (*e.g.*, ligation of complementary restriction sites on the antibody gene fragment and vector, or blunt end ligation if no restriction sites are present). Prior to insertion of the antibody or antibody-related light or heavy chain sequences, the expression vector may already carry antibody constant region sequences. For example, one approach to converting an antibody or antibody-related VH and VL sequences to full-length antibody genes is to insert them into expression vectors already encoding heavy chain constant and light chain constant regions, respectively, such that the VH segment is operatively linked to the CH segment(s) within the vector and the VL segment is operatively linked to the CL segment within the vector. Additionally or alternatively, the recombinant expression vector can encode a signal peptide that facilitates production of the antibody chain from a host cell. The antibody chain gene can be cloned into the vector such that the signal peptide is linked in-frame to the amino terminus of the antibody chain gene. The signal peptide can be an immunoglobulin signal peptide or a heterologous signal peptide (*i.e.,* a signal peptide from a non-immunoglobulin protein).

In addition to the antibody chain genes, a recombinant expression vector can carry one or more regulatory sequence that controls the expression of the antibody chain genes in a host cell. The term "regulatory sequence" is intended to include promoters, enhancers and other expression control elements (*e.g.*, polyadenylation signals) that control the transcription or translation of the antibody chain genes. Such regulatory sequences are described, *e.g*., in Goeddel; Gene Expression Technology: Methods in Enzymology 185, Academic Press, San Diego, CA (1990): It will be appreciated by those skilled in the art that the design of the expression vector, including the selection of regulatory sequences may depend on such factors as the choice of the host cell to be transformed, the level of expression of protein desired, etc. Suitable regulatory sequences for mammalian host cell expression include viral elements that direct high levels of protein expression in mammalian cells, such as promoters and/or enhancers derived from cytomegalovirus (CMV) (such as the CMV promoter/enhancer), Simian Virus 40 (SV40) (such as the SV40 promoter/enhancer), adenovirus, (*e.g.*, the adenovirus major late promoter (AdMLP)) and polyoma. For further description of viral regulatory elements, and sequences thereof, see, *e.g.,* U.S. Patent No. 5,168,062 by Stinski, U.S. Patent No. 4,510,245 by Bell et al. and U.S. Patent No. 4,968,615 by Schaffner et al.

In addition to the antibody chain genes and regulatory sequences, a recombinant expression vector may carry one or more additional sequences, such as a sequence that regulates replication of the vector in host cells (*e.g.*, origins of replication) and/or a selectable marker gene. The selectable marker gene facilitates selection of host cells into which the vector has been introduced (see *e.g.,* U.S. Patents Nos. 4,399,216, 4,634,665 and 5,179,017, all by Axel et al.).

For example, typically the selectable marker gene confers resistance to drugs, such as G418, hygromycin or methotrexate, on a host cell into which the vector has been introduced. Suitable selectable marker genes include the dihydrofolate reductase (DHFR) gene (for use in dhfr- host cells with methotrexate selection/amplification) and the neo gene (for G418 selection).

An antibody, or antibody portion, of the present disclosure can be prepared by recombinant expression of immunoglobulin light and heavy chain genes in a host cell, for example, a CHO cell. To express an antibody recombinantly, a host cell is transfected with one or more recombinant expression vectors carrying DNA fragments encoding the immunoglobulin light and heavy chains of the antibody such that the light and heavy chains are expressed in the host cell and secreted into the medium in which the host cells are cultured, from which medium the antibodies can be recovered. Standard recombinant DNA methodologies are used to obtain antibody heavy and light chain genes, incorporate these genes into recombinant expression vectors and introduce the vectors into host cells, such as those described in Sambrook, Fritsch and Maniatis (eds), Molecular Cloning; A Laboratory Manual, Second Edition, Cold Spring Harbor, N.Y., (1989), Ausubel et al. (eds.) Current Protocols in Molecular Biology, Greene Publishing Associates, (1989) and in U.S. Patent Nos. 4,816,397 & 6,914,128.

For expression of the light and heavy chains, the expression vector(s) encoding the heavy and light chains is (are) transfected into a host cell by standard techniques. The various forms of the term "transfection" are intended to encompass a wide variety of techniques commonly used for the introduction of exogenous DNA into a prokaryotic or eukaryotic host cell, e.g., electroporation, calcium-phosphate precipitation, DEAE-dextran transfection and the like. Although it is theoretically possible to express the antibodies in either prokaryotic or eukaryotic host cells, expression of antibodies in eukaryotic cells, such as mammalian host cells, is suitable because such eukaryotic cells, and in particular mammalian cells, are more likely than prokaryotic cells to assemble and secrete a properly folded and immunologically active antibody. Prokaryotic expression of antibody genes has been reported to be ineffective for production of high yields of active antibody (Boss and Wood (1985) Immunology Today 6:12-13).

Suitable host cells for cloning or expressing the DNA in the vectors herein are prokaryote, yeast, or higher eukaryote cells. Suitable prokaryotes for this purpose include eubacteria, such as Gram-negative or Gram-positive organisms, *e.g.*, Enterobacteriaceae such as Escherichia, *e.g.*, E. coli, Enterobacter, Erwinia, Klebsiella, Proteus, Salmonella, *e.g.,* Salmonella typhimurium, Serratia, *e.g.,* Serratia marcescans, and Shigella, as well as Bacilli such as B. subtilis and B. licheniformis (*e.g.,* B. licheniformis 41P disclosed in DD 266,710 published Apr. 12, 1989), Pseudomonas such as P. aeruginosa, and Streptomyces. One suitable E. coli cloning host is E. coli 294 (ATCC 31,446), although other strains such as E. coli B, E. coli X1776 (ATCC 31,537), and E. coli W3110 (ATCC 27,325) are suitable. These examples are illustrative rather than limiting.

In addition to prokaryotes, eukaryotic microbes such as filamentous fungi or yeast are suitable cloning or expression hosts for polypeptide encoding vectors. Saccharomyces cerevisiae, or common baker's yeast, is the most commonly used among lower eukaryotic host microorganisms. However, a number of other genera, species, and strains are commonly available and useful herein, such as Schizosaccharomyces pombe; Kluyveromyces hosts such as, *e.g.,* K. lactis, K. fragilis (ATCC 12,424), K. bulgaricus (ATCC 16,045), K. wickeramii (ATCC 24,178), K. waltii (ATCC 56,500), K. drosophilarum (ATCC 36,906), K. thermotolerans, and K. marxianus; yarrowia (EP 402,226); Pichia pastoris (EP 183,070); Candida; Trichoderma reesia (EP 244,234); Neurospora crassa; Schwanniomyces such as Schwanniomyces occidentalis; and filamentous fungi such as, *e.g.,* Neurospora, Penicillium, Tolypocladium, and Aspergillus hosts such as A. nidulans and A. niger.

Suitable host cells for the expression of glycosylated antibodies are derived from multicellular organisms. Examples of invertebrate cells include plant and insect cells. Numerous baculoviral strains and variants and corresponding permissive insect host cells from hosts such as Spodoptera frugiperda (caterpillar), Aedes aegypti (mosquito), Aedes albopictus (mosquito), Drosophila melanogaster (fruitfly), and Bombyx mori have been identified. A variety of viral strains for transfection are publicly available, *e.g.*, the L-1 variant of Autographa californica NPV and the Bm-5 strain of Bombyx mori NPV, and such viruses may be used as the virus herein according to the present invention, particularly for transfection of Spodoptera frugiperda cells. Plant cell cultures of cotton, corn, potato, soybean, petunia, tomato, and tobacco can also be utilized as hosts.

Suitable mammalian host cells for expressing recombinant antibodies include Chinese Hamster Ovary (CHO cells) (including dhfr- CHO cells, described in Urlaub and Chasin, (1980) PNAS USA 77:4216-4220, used with a DHFR selectable marker, *e.g.*, as described in Kaufman and Sharp (1982) Mol. Biol. 159:601-621),

NS0 myeloma cells, COS cells and SP2 cells. When recombinant expression vectors encoding antibody genes are introduced into mammalian host cells, the antibodies are produced by culturing the host cells for a period of time sufficient to allow for expression of the antibody in the host cells or production of the antibody into the culture medium in which the host cells are grown. Other examples of useful mammalian host cell lines are monkey kidney CV1 line transformed by SV40 (COS-7, ATCC CRL 1651); human embryonic kidney line (293 or 293 cells subcloned for growth in suspension culture, Graham et al., J. Gen Virol. 36:59 (1977)); baby hamster kidney cells (BHK, ATCC CCL 10); Chinese hamster ovary cells/-DHFR (CHO, Urlaub et al., Proc. Natl. Acad. Sci. USA 77:4216 (1980)); mouse sertoli cells (TM4, Mather, Biol. Reprod. 23:243-251 (1980)); monkey kidney cells (CV1 ATCC CCL 70); African green monkey kidney cells (VERO-76, ATCC CRL-1587); human cervical carcinoma cells (HELA, ATCC CCL 2); canine kidney cells (MDCK, ATCC CCL 34); buffalo rat liver cells (BRL 3A, ATCC CRL 1442); human lung cells (W138, ATCC CCL 75); human liver cells (Hep G2, HB 8065); mouse mammary tumor (MMT 060562, ATCC CCL51); TRI cells (Mather et al., Annals N.Y. Acad. Sci. 383:44-68 (1982)); MRC 5 cells; FS4 cells; and a human hepatoma line (Hep G2).

Host cells are transformed with the above-described expression or cloning vectors for antibody production and cultured in conventional nutrient media modified as appropriate for inducing promoters, selecting transformants, or amplifying the genes encoding the desired sequences.

The host cells used to produce an antibody may be cultured in a variety of media. Commercially available media such as Ham's F10™ (Sigma), Minimal Essential Medium™ ((MEM), (Sigma), RPMI-1640 (Sigma), and Dulbecco's Modified Eagle's Medium™ ((DMEM), Sigma) are suitable for culturing the host cells. In addition, any of the media described in Ham et al., Meth. Enz. 58:44 (1979), Barnes et al., Anal. Biochem. 102:255 (1980), U.S. Pat. Nos. 4,767,704; 4,657,866; 4,927,762; 4,560,655; or 5,122,469; WO 90/03430; WO 87/00195; or U.S. Pat. No. Re. 30,985 may be used as culture media for the host cells.

Any of these media may be supplemented as necessary with hormones and/or other growth factors (such as insulin, transferrin, or epidermal growth factor), salts (such as sodium chloride, calcium, magnesium, and phosphate), buffers (such as HEPES), nucleotides (such as adenosine and thymidine), antibiotics (such as gentamycin drug), trace elements (defined as inorganic compounds usually present at final concentrations in the micromolar range), and glucose or an equivalent energy source. Any other necessary supplements may also be included at appropriate concentrations that would be known to those skilled in the art. The culture conditions, such as temperature, pH, and the like, are those previously used with the host cell selected for expression, and will be apparent to the ordinarily skilled artisan.

Host cells can also be used to produce portions of intact antibodies, such as Fab fragments or scFv molecules. It is understood that variations on the above procedure are within the scope of the present invention. For example, in certain embodiments it may be desirable to transfect a host cell with DNA encoding either the light chain or the heavy chain (but not both) of an antibody of this disclosure. Recombinant DNA technology may also be used to remove some or all of the DNA encoding either or both of the light and heavy chains that is not necessary for binding to a target antigen. The molecules expressed from such truncated DNA molecules are also encompassed by the antibodies of the present disclosure. In addition, bifunctional antibodies may be produced in which one heavy and one light chain are from a first antibody and the other heavy and light chain are specific for a different antigen, by crosslinking a first antibody to a second antibody by standard chemical crosslinking methods.

In a suitable system for recombinant expression of an antibody, or antigen-binding portion thereof, a recombinant expression vector encoding both the antibody heavy chain and the antibody light chain is introduced into dhfr-CHO cells by calcium phosphate-mediated transfection. Within the recombinant expression vector, the antibody heavy and light chain genes are each operatively linked to CMV enhancer/AdMLP promoter regulatory elements to drive high levels of transcription of the genes. The recombinant expression vector also carries a DHFR gene, which allows for selection of CHO cells that have been transfected with the vector using methotrexate selection/amplification. The selected transformant host cells are cultured to allow for expression of the antibody heavy and light chains and intact antibody is recovered from the culture medium. Standard molecular biology techniques are used to prepare the recombinant expression vector, transfect the host cells, select for transformants, culture the host cells and recover the antibody from the culture medium.

When using recombinant techniques, the antibody can be produced intracellularly, in the periplasmic space, or directly secreted into the medium. In certain embodiments, if the antibody is produced intracellularly, as a first step, the particulate debris, either host cells or lysed cells (*e.g.*, resulting from homogenization), can be removed, *e.g.*, by centrifugation or ultrafiltration. Where the antibody is secreted into the medium, supernatants from such expression systems can be first concentrated using a commercially available protein concentration filter, *e.g.*, an Amicon™ or Millipore Pellicon™ ultrafiltration unit.

### 3. Large Scale Antibody Production Incorporating Tensegrity Force Optimization

In certain embodiments, the tensegrity model can be scaled-up such that tensegrity forces can be applied to cells producing commercially relevant antibodies, for example, cells cultured in commercial bioreactors.

In certain embodiments, mechanical shear stress can be applied to cells cultured in a bioreactor. For example, such stress can be applied to the cells via vortexing or mixing the cells at a rate effective to induce a mechanical stress, for example, stretching of a cells membrane. In certain embodiments, such mechanical stress is effective to increase production of antibody from the cells in culture.

In certain embodiments, the cells cultured in a bioreactor are contacted with a magnetic bead, for example, a ferromagnetic microbead as described previously, wherein shear stress (torque) can be applied to the surface using membrane-bound magnetic beads in an amount effective to increase antibody production from the cells.

In certain embodiments, tensegrity force can be applied to cells cultured in a bioreactor, for example, by a biomechanical system capable of subjecting cells to diverse biaxial stress/strain. For example, the bioreactor can comprise a substrate for the cells of the culture to adhere to. Such a substrate can include a plurality of surface areas to which the cells adhere to. In certain embodiments, the substrate can comprise a honeycomb-like substrate to which the cells adhere to. In certain embodiments, the bioreactor includes beads or other substrates to which the cells can adhere to. In certain embodiments, the substrate can be stretched or manipulated along one or more axis in an amount effective to change the morphology or shape of the cells adhered to the substrate. In certain embodiments, the morphology or shape of the cells is altered in an amount effective to increase the production of antibody from the cells.

In certain embodiments, cells cultured in a bioreactor are subjected to tensegrity forces by applying cyclic sound pressure to the cells. For example, ultrasound waves, or any other high or low frequency sound waves, can be applied to the cells in an amount effective to increase the production of antibody from the cells. In certain embodiments, the cells are free floating cells.

In certain embodiments, tensegrity forces can produce long-lasting or permanent changes in properties of cells or cell culture, for example, an increase in antibody production by the cells. Such changes in cell properties can be caused by a modification in gene expression. For example, but not by way of limitation, cyclic stretching of adherent CHO cells on plate cultures can result in a permanent increase in antibody expression and/or secretion by the cells. These cells with increased antibody expression and/or secretion can then be scaled up in bioreactors where the cells are grown in suspension.

### EXAMPLES

### 1. Cell Culture

CHO cells producing recombinant, glycosylated antibody (e.g., ABT-874) are used. Floating CHO cells producing ABT-874 are adapted to growth in in serum. The cells are cultured on cell culture plates at nominal growth conditions used in the fed batch reactor

### 2 Creation of tensegrity forces on the cells

The cells are introduced to the tensegrity model using two different techniques described below in sections 2.1 and 2.2. The mechanotransduction changes induced by these forces are optimized and evaluated using cell based assays.

### 2.1 Optical Magnetic Twisting Cytometry

At varied time points, the cells are subjected to a controlled mechanical shear stress, applied directly to cell surface. Shear stress (torque) is applied to the surface using membrane-bound ferromagnetic beads (1-6 µm in diameter) coated with anti-CHO antibodies that adhere to the cytoskeleton of the cell. The beads are magnetized in one direction by applying a weaker twisting magnetic field. Electron microscopy images are taken to show ferromagnetic bead attachment to the surface of CHO cells.

The cellular deformation that results in response to stress application is determined using magnetic twisting cytometry (MTC). MTC is a technique used to exert mechanical stresses on living cells by first magnetizing and then rotating ferro magnetic beads that are bound to the surface of cells. The experimental procedure and the parameters for applying the magnetic field is as described in the protocol by Na. S and Wang. N (2008) Science Signaling 1(34):pll. Briefly, the MTC device consists of seven major components:(i) a high-voltage generator to provide the current to magnetize the beads; (ii) one [for one-dimensional (1D) MTC] bipolar current sources for twisting the beads; (iii) a computer for controlling the twisting apparatus; (iv) an inverted microscope for observing the sample; (v) a charge-coupled device (CCD) camera that uses software capable of synchronizing image capture with step function or oscillatory wave magnetic fields; (vi) a device to maintain the correct temperature of the cultured cells; and (vii) a microscope insert that holds the sample and contains two pairs of coils for 1D MTC that generate the alternating electric fields used to magnetize and twist the beads (available commercially from Eberhard EOL Inc., Switzerland). Na. S and Wang.N (2008) Science Signaling 1(34):p11.

### 2.2 Traction microscopy

Traction microscopy is a biomechanical culture system capable of subjecting adherent cells to diverse biaxial stress/strain culture conditions and test while permitting live microscopic imaging. The cells are attached on a hydrogel substrate like polyacrylamide, which is further coated with collagen. 0.2 µm diameter fluorescent microbeads beads are embedded near the gel apical surface to trace the dynamics of the cytoskeleton network under the microscope. An, S.S., et al., (2006) Am J Respir Cell Mol Biol 35(1): p. 55-64. The stiffness and the stretch are the two controlled variables in the system. Stiffness is controlled by adjusting the concentration of the hydrogel substrate and the stretch forces are controlled by the magnitude of the forces applied. The hydrogel is stretched symmetrically along two orthogonal axes using computer controlled stepper motors. This arrangement allows control of multiple parameters including the strain magnitude and strain rate. Artmann, G.M. and S. Chien, (2008), Bioengineering in cell and tissue research. Springer.

The hydrogel substrate is stretched at an interval of about 20 seconds for about 30 minutes. The changes in morphology are captured by fluorescence imaging. The cells are further incubated and the changes due to mechanotransduction are measured at various time points by performing cell based assays.

### 3. Quantitative analysis of cell viability

Calcein AM is a widely used green fluorescent cell marker. Calcein AM is membrane-permeant and can be introduced into cells via incubation. Once inside the cells, Calcein AM (a nonfluorescent molecule) is hydrolyzed by endogenous esterase into the highly negatively charged green fluorescent calcein. The fluorescent calcein is retained in the cytoplasm in live cells. Calcein AM serves as an excellent tool for the studies of cell membrane integrity and for long-term cell tracing due to its lack of cellular toxicity. The cells are further quantified by obtaining fluorescence microscopy images using a phase contrast microscope.

### 4. Qualitative analysis of cell viability 4.1 XTT assay

XTT Cell Viability Assay Kit provides a simple method for determination of live cell number using standard microplate absorbance readers. Determination of live cell number is often used to assess rate of cell proliferation and to screen cytotoxic agents. XTT is a tetrazolium derivative. It measures cell viability based on the activity of mitochondria enzymes in live cells that reduce XTT and are inactivated shortly after cell death. On incubation, it is readily reduced to a highly water-soluble orange colored product. The amount of water-soluble product generated from XTT is proportional to the number of living cells in the sample and is quantified by measuring absorbance at wavelength of 475 nm.

### 4.2 Trypan Blue Exclusion Test of Cell Viability

The dye exclusion test is used to determine the number of viable cells present in a cell suspension. It is based on the principle that live cells possess intact cell membranes that exclude certain dyes, such as trypan blue, Eosin, or propidium, whereas dead cells do not. In this test, a cell suspension is mixed with dye and then visually examined to determine whether cells take up or exclude dye. The cells are counted using a hemacytometer mounted on an inverted light microscope. Viable cells are distinguished due to their clear cytoplasm, whereas nonviable cells have a blue cytoplasm.

### 5. Production of IgG

The cells used in the instant study secrete IgG. The IgG is released directly into the media that forms the cell's microenvironment. Applied Biosystems ImmunoDetection Sensor Cartridge is an assay device for measurement of most subclasses of IgG (Immunoglobulin G) from various animal species. It contains a Protein A ligand covalently bound to the surface of a macroporous polymer resin. The cells are centrifuged and the supernatant collected is injected on Protein A column. IgG in the sample is captured and concentrated in the sensor cartridge. Bound IgG is then eluted from the sensor cartridge by lowering the Mobile Phase pH and detecting the eluted material directly at 280nm. This procedure is applicable to concentration determination of interested IgG in mammalian cell culture based fermentation samples.

### 6. Cell Morphology

Variations in cell migration and morphology are consequences of changes in underlying cytoskeletal organization and dynamics. It is useful to understand the changes in cell structures due to application of external forces. Progress of a cell through its life cycle is based on a continuum of causally related cellular events. State of the art microscopic techniques like Scanning Electron Microscopy, phase contrast microscopy; transmission electron microscopy are used to evaluate the changes in the cell behavior over time

## Claims

1. A method for increasing production of an antibody of interest from a host cell comprising subjecting said cell to a tensegrity force, wherein the tensegrity force is applied to the cell in an amount effective to increase said antibody production from the cell.

2. The method of claim 1, wherein the tensegrity force is a mechanical shear stress.

3. The method of claim 1, wherein the cell is adherent to a substrate and the tensegrity force is a biaxial stress.

4. The method of claim 1, wherein the cell is a Chinese hamster ovary (CHO) cell.

5. The method of claim 1, wherein the antibody is ABT-874.

6. The method of claim 1, wherein the cell is cultured in a bioreactor.

7. The method of claim 7, wherein the cell is adhered to a substrate.

8. The method of claim 1, wherein the tensegrity force is cyclic sound pressure.

9. The method of claim 8, wherein the cyclic sound pressure is ultrasound.

10. The method of claim 2, wherein the cell is contacted with a ferromagnetic microbead, and wherein a magnetic force is applied to the ferromagnetic microbead.

## Patentansprüche

1. Ein Verfahren zur Erhöhung der Produktion eines Antikörpers von Interesse von einer Wirtszelle umfassend das Unterwerfen der Zelle gegenüber einer Tensegrity Kraft, worin die Tensegrity Kraft an die Zelle angebracht wird in einer Menge, die wirksam ist, um die Antikörperproduktion von der Zelle zu erhöhen.

2. Das Verfahren gemäß Anspruch 1, worin die Tensegrity Kraft eine mechanische Scherbeanspruchung ist.

3. Das Verfahren gemäß Anspruch 1, worin die Zelle an ein Substrat anhaftend ist und die Tensegrity Kraft ist eine biaxiale Beanspruchung.

4. Das Verfahren gemäß Anspruch 1, worin die Zelle eine chinesische Hamster Ovarial (CHO)-Zelle ist.

5. Das Verfahren gemäß Anspruch 1, worin der Antikörper ABT-874 ist.

6. Das Verfahren gemäß Anspruch 1, worin die Zelle in einem Bioreaktor kultiviert wird.

7. Das Verfahren gemäß Anspruch 7, worin die Zelle an ein Substrat angeheftet ist.

8. Das Verfahren gemäß Anspruch 1, worin die Tensegrity Kraft ein periodischer Schaltdruck ist.

9. Das Verfahren gemäß Anspruch 8, worin der periodische Schalldruck Ultraschall ist.

10. Das Verfahren gemäß Anspruch 2, worin die Zelle mit einem ferromagnetischen Mikrokügelchen in Kontakt gebracht wird, und worin eine magnetische Kraft auf das ferromagnetische Mikrokügelchen angebracht wird.

## Revendications

1. Procédé pour augmenter la production d'un anticorps d'intérêt par une cellule hôte comprenant l'exposition de ladite cellule à une force de tenségrité, où la force de tenségrité est appliquée à la cellule en une quantité efficace pour augmenter ladite production d'anticorps par la cellule.

2. Procédé selon la revendication 1, où la force de tenségrité est une contrainte de cisaillement mécanique.

3. Procédé selon la revendication 1, où la cellule est adhérente à un substrat et la force de tenségrité est une contrainte biaxiale.

4. Procédé selon la revendication 1, où la cellule est une cellule d'ovaire de hamster chinois (CHO).

5. Procédé selon la revendication 1, où l'anticorps est ABT-874.

6. Procédé selon la revendication 1, où la cellule est cultivée dans un bioréacteur.

7. Procédé selon la revendication 7, où la cellule est amenée à adhérer à un substrat.

8. Procédé selon la revendication 1, où la force de tenségrité est une pression sonore cyclique.

9. Procédé selon la revendication 8, où la pression sonore cyclique est un ultrason.

10. Procédé selon la revendication 2, où la cellule est mise en contact avec une microbille ferromagnétique, et où une force magnétique est appliquée à la microbille ferromagnétique.
